(19) Europäisches Patentamt
European Patent Office
Office européen des brevets

(11) Veröffentlichungsnummer: **0 269 999 B1**

(12) **EUROPÄISCHE PATENTSCHRIFT**

(45) Veröffentlichungstag der Patentschrift: **22.05.91**

(51) Int. Cl.⁵: **C07C 31/125**, C07C 69/14, C07C 33/025, C07C 69/24, C07C 69/145, C07C 29/14, C07C 67/08, A61K 7/46

(21) Anmeldenummer: **87117465.2**

(22) Anmeldetag: **26.11.87**

(54) **Neue aliphatische Alkohole und Ester, deren Herstellung und Verwendung als Riechstoffe.**

(30) Priorität: **03.12.86 DE 3641236**

(43) Veröffentlichungstag der Anmeldung:
**08.06.88 Patentblatt 88/23**

(45) Bekanntmachung des Hinweises auf die Patenterteilung:
**22.05.91 Patentblatt 91/21**

(84) Benannte Vertragsstaaten:
**CH DE ES FR GB LI NL**

(56) Entgegenhaltungen:
**DE-A- 3 245 047**

(73) Patentinhaber: **BASF Aktiengesellschaft**
**Carl-Bosch-Strasse 38**
**W-6700 Ludwigshafen(DE)**

(72) Erfinder: **Gramlich, Walter, Dr.**
**Auf der Hoehe 11**
**W-6803 Edingen-Neckarhausen(DE)**
Erfinder: **Siegel, Hardo, Dr.**
**Hans-Purrmann-Allee 25**
**W-6720 Speyer(DE)**

EP 0 269 999 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue aliphatische Alkohole und Ester der Formel I

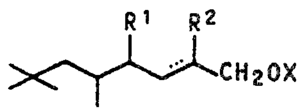

(I)

in der

$R^1$ für Wasserstoff oder eine Methyl-Gruppe,

$R^2$ für einen geradkettigen oder verzweigten $C_1$-$C_4$-Alkyl-Rest,

X für Wasserstoff oder eine Acylgruppe mit 2 bis 4 C-Atomen steht und die gepunktete Linie für eine weitere CC-Bindung stehen kann.

Die Erfindung betrifft ferner Verfahren zur Herstellung der neuen Verbindungen sowie deren Verwendung als Riechstoffe bzw. Bestandteil von Parfümkompositionen, d.h. zur Verleihung, Verbesserung oder Modifizierung der Dufteigenschaften von Parfüms oder parfümierten Produkten.

Trotz der großen Zahl bereits bekannter synthetischer Riechstoffe besteht auf dem Gebiet der Parfümerie ein laufender Bedarf an neuen Riechstoffen, die entweder neue Geruchsnoten aufweisen, teure oder schwer zugängliche Riechstoffe ersetzen können, sowie aus gut verfügbaren und wenig Umweltbelastenden Vorprodukten hergestellt werden können.

Auf der Suche nach preiswerten, gut zugänglichen Riechstoffen mit interessanten Geruchsnoten, die nach wenig Umwelt-belastenden Verfahren hergestellt werden können, wurden die erfindungsgemäßen neuen Verbindungen gefunden.

Es existieren bereits einige Verbindungen mit einer gewissen strukturellen Beziehung zu den erfindungsgemäßen Verbindungen der Formel I, wie z.B:

A. 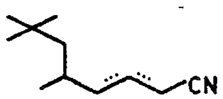

5,7,7-Trimethyl-2-octennitril
(vgl. US 3 531 510)

B. 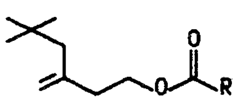

Ester von
3-Methylen-5,5-dimethyl-hexanol
(vgl. DE-OS 2108 805)

C. 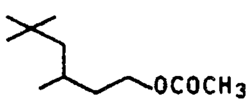

Vanoris®
(International Flavors & Fragrances Inc.)

D. 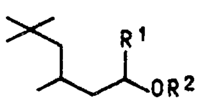

(vgl. DE-OS 28 04 167; BASF AG;
$R^1$ = $C_1$-$C_4$-Alkyl-, Alkenyl-Rest
$R^2$ = Wasserstoff, $C_1$-$C_4$-Acyl-Rest)

E. 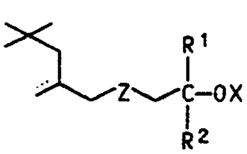

(vgl. DE-OS 32 45 047; Firmenich S.A., Genf;
Z = Ethylen-, Ethenyl-, Ethinyl-Rest
R = Wasserstoff, Alkyl-Rest
X = Wasserstoff, Acyl-Rest)

Die Verbindungen von Gruppe A besitzen einen Geruch, der an Gewürznelke oder Iris erinnert mit erdiger, frischer und grüner Note. Der Geruch der Ester von Gruppe B hängt vom Substituenten R ab und schwankt von pfefferminzartig bis blumig, Irisähnlich, fruchtig oder holzig. Vanoris, ein Verkaufsprodukt der

Fa. International Flavors & Fragrances (C) weist eine fruchtige Holznote mit einer Nuance, die an Schwertlilie erinnert, auf, daneben ein Veilchen-Iononartiges Aroma.

Die Verbindungen der Gruppe D weisen je nach den Resten $R^1$ und $R^2$ Grün-Noten ($R^1$ = Methylgruppe) bis fruchtige Noten ($R^1$ = Vinyl, Ethyl) auf.

Von den Alkoholen und Estern der Gruppe E stellt 5,5,7-Trimethyl-octylpropionat ($R^1$ = $R^2$ = H, Z = $CH_2CH_2$, X = -COEt) eine besonders wertvolle Verbindung dar (vgl. Anwendungsbeispiele in DE-OS 32 45 047), die eine fruchtige, birnenähnliche Note aufweist.

Die erfindungsgemäßen Verbindungen (I) besitzen wertvolle Dufteigenschaften, die sich von den bekannten, stukturell verwandten Verbindungen A - E eindeutig unterscheiden. Währen die bekannten Verbindungen überwiegend blumige und fruchtige Duftnoten aufweisen, zeigen die erfindungsgemäßen Verbindungen in der Parfümerie äußerst begehrte Sandelholz-Noten. Aufgrund ihrer Geruchseigenschaften sowie ihrer guten Verfügbarkeit können die neuen Verbindungen in einer Vielzahl verschiedener Kompositionen eingesetzt werden.

Die bevorzugten Verbindungen enthalten für den Rest X Wasserstoff, eine Acetyl- oder eine Propionyl-Gruppe, Für $R^1$ Wasserstoff und für $R^2$ eine Methylgruppe.

Zwar unterscheidet sich z. B. das erfindungsgemäße 2,5,7,7-Tetramethyl-octylpropionat nur um eine Methylgruppe in 2-Stellung von dem in der DE-OS 32 45 047 als interessant beschriebenen 5,7,7-Trimethyl-octylpropionat, jedoch sind die olfaktorischen Eigenschaften extrem verschieden. Während das bekannte 5,7,7-Trimethyl-octylpropionat eine fruchtige Birnen-ähnliche Note besitzt, weist das erfindungsgemäße 2,5,7,7-Tetramethyl-octylpropionat einen wertvollen Sandelholz-Duft mit einer Vetiver-Note und schwach erdigen Tönen auf; das entsprechende 2,5,7,7-Tetramethyl-octylacetat hingegen besitzt einen frischen Blumenduft ohne ihgendwelche fruchtige Noten. Darüberhinaus können die erfindungsgemäßen Alkohole mit der offensichtlich für den besonderen Geruch entscheidenden Alkylgruppe in $\alpha$-Stellung zur Methylolgruppe nach dem in der DE-OS 32 45 047 beschriebenen Verfahren gar nicht erhalten werden.

Die Herstellung der erfindungsgemäßen Verbindungen erfolgt durch selektive oder vollständige Reduktion bzw. Hydrierung der in der nicht vorveröffentlichten DE-OS 36 22 600 beschriebenen Octenale bzw. Octanale der allgemeinen Formel II

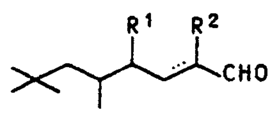

(II)

und gegebenenfalls anschließende Veresterung in an sich bekannter Weise, so daß sich detaillierte Angaben hierüber erübrigen. Im nachfolgenden Schema 1 werden die Synthesemöglichkeiten aufgezeigt und diese anschließend kurz erläutert.

Schema 1

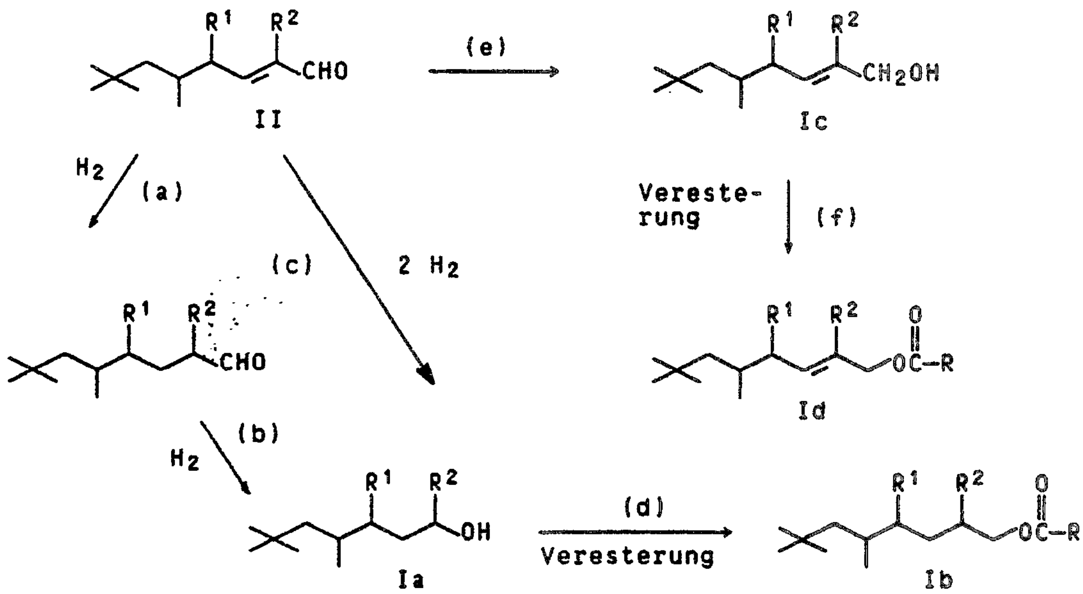


Erläuterungen zu Schema 1:

Für den selektiven Hydrierschritt (a) haben sich am besten Palladium-Katalysatoren auf verschiedenen Trägern bewährt.

Will man zu den entsprechenden gesättigten Alkoholen Ia gelangen, so kann man entweder nach (b) das erhaltene gesättigte Octanal erneut katalytisch hydrieren, oder aber man hydriert den ungesättigten Aldehyd II direkt zum gesättigten Alkohol (Weg c).

Als Hydrierkatalysatoren eignen sich nahezu alle Edelmetallkatalysatoren; bevorzugt werden Platin, Ruthenium auf Kohle-Trägern oder Raney-Nickel.

Die erhaltenen gesättigten Alkohole Ia lassen sich dann nach bekannten Methoden zu den gesättigten Estern Ib verestern (Weg d); am besten hat sich hier die Umsetzung mit den entsprechenden Säureanhydriden bewährt.

Die durch Aldolkondensation erhaltenen Octenale II lassen sich aber auch unter Erhalt der CC-Doppelbindung an der Aldehyd-Funktion zu den ungesättigten Alkoholen Ic reduzieren (Weg e). Die Reduktion kann sowohl nach Meerwein-Ponndorf-Verley mit Aluminium-isopropylat, als auch mit speziellen Hydrierkatalysatoren (vgl. P.N. Rylander, Catalytic Hydrogenation in Organic Synthesis, Academic Press New York 1979, Seiten 74 bis 76) erfolgen.

Die erhaltenen ungesättigten Alkohole Ic lassen sich danach ebenfalls zu den ungesättigten Estern Id verestern (Weg f).

Als Ester sind Formiate, Acetate, Propionate, Butyrate oder Isobutyrate von Interesse; bevorzugt werden die Acetate und die Propionate.

Ein ganz wesentlicher Vorteil der vorliegenden Erfindung im Vergleich zu dem Gegenstand der zitierten DE-OS 32 45 047 liegt neben den wertvollen olfaktorischen Eigenschaften in dem überlegenen technischen Fortschritt des Herstellverfahrens. Bei dem erfindungsgemäßen Verfahren werden beide Reaktionsstufen katalytisch durchgeführt. Demgegenüber fallen bei dem in loc.cit. beschriebenen Verfahren molare Mengen an Alkalihalogeniden an. Darüberhinaus wird $Cu_2Cl_2$ als Katalysator eingesetzt, was zu einer weiteren Umwelt-Belastung mit Schwermetallen führt. Außerdem sind die gemäß diesem Verfahren erzielten Ausbeuten von weniger als 50 % für ein technisches Verfahren unbefriedigend.

Die erfindungsgemäßen Verbindungen haben neben ihrer Bedeutung als Riechstoffe durch ihre Struktur Interesse als Zwischenprodukte für weitere Synthesen, insbesondere zur Herstellung anderer interessanter Riechstoffe, gefunden. Sie lassen sich sehr gut mit den üblichen Parfüminhaltsstoffen und anderen Riechstoffen zu neuartigen Kompositionen kombinieren.

Der Anteil der erfindungsgemäßen Verbindungen in den Riechstoffkompositionen beträgt im allgemeinen 1 bis 50 Gew%. Die auf diese Weise hergestellten Kompositionen können entweder direkt in der Fein-Parfümerie eingesetzt werden, oder aber zur Parfümierung von kosmetischen Präparaten wie Cremes, Lotionen, Duftwässern, Aerosolen, Toilettenseifen, Mundpflegemitteln sowie zur Geruchsverbesserung von technischen Produkten wie Weichspülern, Wasch- und Reinigungsmitteln sowie Desinfektionsmitteln verwendet werden. Die folgenden Beispiele sollen den Gegenstand der Erfindung näher erläutern, ohne ihn jedoch hierauf zu beschränken.

Beispiel 1

2,5,7,7-Tetramethyl-octanol

In einem 1,2 l-Autoklaven wurden 500 g (2,7 mol) 2,5,7,7-Tetramethyl-octanal in Gegenwart von 40 g Raney-Nickel zunächst 5 Stunden (h) bei einer Temperatur von 100 °C und 60 bar Wasserstoffdruck und dann bei 150 °C und 200 bar Wasserstoffdruck bis zur Druckkonstanz (ca. 5 h) hydriert. Nach Abtrennen des Katalysators wurde durch fraktionierte Destillation gereinigt. Man erhielt 486 g (2,6 mol) 2,5,7,7-Tetramethyl-octanol (96 % Ausbeute), Kp. 70 °C/0.15 mbar; $n_D^{25}$ : 1.4400
IR: 3335, 2955, 2914, 2870, 1476, 1466, 1393, 1377, 1384, 1088 $cm^{-1}$.
$^1$H-NMR($CDCl_3$): $\delta$ = 0,89(9H,s), 0,92(6H,dd), 1,0 - 1,7(9H, m), 3,45(2H, m), ppm.
MS: $M^+$ = 186(CI-MS); m/e(%): 171(1,5), 129(4),112(5,5), 97(12), 83(7,5), 69(15), 57(100), 41(23), 29(12), 18-(15). $C_{11}H_{26}O$ (186)

Die Verbindung weist einen blumigen, Rosen-artigen Duft auf.

Beispiel 2

2,5,7,7-Tetramethyl-octylacetat

Zu einem Gemisch bestehend aus 153 g (1,5 mol) Acetanhydrid und 1 g Phosphorsäure tropfte man innerhalb von 15 bis 30 Minuten (min) bei 50 bis 60 °C unter leichter Kühlung 152 g (0,8 mol) 2,5,7,7-Tetramethyl-octanol zu und rührte dann das Reaktionsgemisch noch 12 h nach.

Nach Abdestillieren eines Gemisches aus Acetanhydrid und Essigsäure wurde mit 250 ml Diethylether aufgenommen, mit wäßriger Natriumhydrogencarbonat-Lösung gewaschen und nach Entfernen des Lösungsmittels der Rückstand fraktioniert destilliert. Man erhielt 152 g (0,66 mol) 2,5,7,7-Tetramethyl-octylacetat als farbloses Öl (Ausbeute 83 %), Kp. 48 °C/0,01 mbar; $n_D^{25}$ : 1.4298
IR: 2956, 1744, 1467, 1392, 1365, 1240, 1037 $cm^{-1}$.
MS: $M^+$ = 228(CI-MS); m/e(%): 213(0,5), 173(1,5), 112(28), 97(23), 83(17), 69(17,5), 57(100), 43(42), 28-(15).
$^1$H-NMR($CDCl_3$) $\delta$ = 0.9(s,9H), 0.93(d, 3H), 1 - 1.5(m, 10H), 1.75(m,1H), 2.05(s,3H), 3,85(m,1H), 3.95(m,1H) ppm. $C_{14}H_{28}O_2$ (228)

Die Verbindung weist einen sehr frischen blumigen Duft auf.

Beispiel 3

2,5,7,7-Tetramethyl-octylpropionat

Zu einem Gemisch aus 130 g (1 mol) Propionsäureanhydrid und 1 g Phosphorsäure tropfte man bei 75 °C 80 g (0,43 mol) von 2,5,7,7-Tetramethyl-octanol und arbeitete analog Beispiel 2 auf. Durch Destillation erhielt man 78 g (75 % Ausbeute) an 2,5,7,7-Tetramethyl-octylpropionat, Kp 66 °C/0,01 mbar; $n_D^{25}$ : 1.4316
IR: 2955, 1742, 1465, 1392, 1378, 1365, 1187, 1083, 1019 $cm^{-1}$.
$^1$H-NMR($CDCl_3$): $\delta$ = 0.9(s,9H), 0.91(6H), 1.17(t,3H), 1 -1.8(m,8H), 2,35(q,2H), 3.8-4.0(m,2H) ppm.
MS : $M^+$ = 242(0.1 %); m/e: 112(7,5), 97(9), 83(6), 70(5.5), 57(72), 43(100).
$C_{15}H_{30}O_2$ (242).

Die Verbindung weist einen interessanten Sandelholz-Duft mit Vetiver-Note sowie einer schwach erdigen Nuance auf.

Beispiel 4

2,5,7,7-Tetramethyl-2-octen-1-ol

Eine Mischung aus 240 g (1.32 mol) 2,5,7,7-Tetramethyl-2-octen-1-al, 1000 ml Isopropanol und 122 g (0,6 mol) Aluminiumisopropylat wurden unter Rückfluß zum Sieden erhitzt und dann bei einer Innentemperatur von 70 bis 75° C das entstandene Aceton abdestilliert.

Nach Reaktionsende wurde das überschüssige Isopropanol abdestilliert, der Rückstand unter Eiskühlung mit 50 gew.%iger Schwefelsäure angesäuert, mit Diethylether extrahiert, die Etherphase einmal neutral gewaschen und nach Entfernen des Lösungsmittels der Rückstand fraktioniert destilliert.

Man erhielt 180 g 2,5,7,7-Tetramethyl-2-octen-1-ol (74 % Ausbeute) mit Kp. 64° C/0,3 mbar; $n_D^{25}$ : 1.4550

IR: 3327, 2953; 1476, 1467, 1393, 1376, 1364, 1011 $cm^{-1}$.

$^1$H-NMR(CDCl$_3$/E : Z = 95 : 5) $\delta$ = 0.92(s,9H), 0.95(s,3H), 1.05(m,1H), 1.25(m,1H), 1.6(br.s,2H/1H=OH), 1.67(s,3H), 1.9(m,1H), 2.0(m,1H), 4.02(s,2H), 5.42(t,1H) ppm.

MS: $M^+$ = 184(3%):; m/e(%): 166(0.5), 110(5), 95(8), 83(7), 68(24), 57(100), 43(35).

$C_{12}H_{24}O$ (184).

Die Verbindung weist einen interessanten Holz-Duft mit einer Kräuternote auf.

Beispiel 5

2,5,7,7-Tetramethyl-2-octenyl-1-acetat

Zu einem Gemisch aus 102 g (1 mol) Acetanhydrid und 1 g Phosphorsäure wurden bei 75° C unter leichtem Kühlen 79 g (0.43 mol) 2,5,7,7-Tetramethyl-2-octen-1-ol zugetropft und das Reaktionsgemisch anschließend noch 12 h gerührt. Durch Aufarbeitung analog Beispiel 2 erhielt man 75 g (77 % Ausbeute) 2,5,7,7-Tetramethyl-2-octen-1-ylacetat.

Kp. 70° C/0,2 mbar; $n_D^{25}$ : 1.4441

IR: 2954, 2907, 2868, 1743, 1466, 1393, 1376, 1365, 1230, 1023 $cm^{-1}$.

$^1$H-NMR: $\delta$ = 0.93(s,9H), 0.95(s,3H), 1.05(m,1H), 1.25(m,1H), 1.58(br.s,1H), 1.65(s,3H), 1.9(m,1H), 2.03-(m,1H), 2.08(s,3H), 4.45(s,2H), 5.48(t,1H) ppm.

MS: $M^+$ 226 (0,5 %); m/e = 184 (1.8), 166 (6.5), 151 (4), 123 (2,8), 110 (15), 95 (18), 83 (10), 68 (67), 57 (100), 43 (66).

Die Verbindung weist eine interessante pudrige Holznote auf.

## Ansprüche

**1.** Aliphatische Alkohole und Ester der allgemeinen Formel I

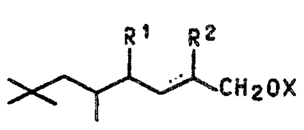

(I)

in der

$R^1$ für Wasserstoff oder eine Methyl-Gruppe,

$R^2$ für einen geradkettigen oder verzweigten $C_1$-$C_4$-Alkyl-Rest, und

X für Wasserstoff oder eine Acyl-Gruppe mit 2 bis 4 C-Atomen steht und die gepunktete Linie für eine weitere C-C-Bindung stehen kann.

**2.** Verbindungen gemäß Anspruch 1, dadurch gekennzeichnet, daß X für eine Acetyl- bzw. Propionyl-Gruppe steht.

**3.** 2,5,7,7-Tetramethyl-octanol

**4.** 2,5,7,7-Tetramethyl-octylacetat

**5.** 2,5,7,7-Tetramethyl-octylpropionat

**6.** 2,5,7,7-Tetramethyl-2-octen-1-ol

**7.** 2,5,7,7-Tetramethyl-2-octen-1-ylacetat

**8.** Verfahren zur Herstellung von aliphatischen Alkoholen und Estern der allgemeinen Formel I, dadurch gekennzeichnet, daß man ein Octenal bzw. Octanal der allgemeinen Formel II

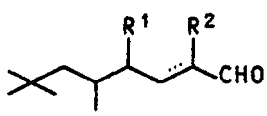

(II)

in der $R^1$ und $R^2$ die im Anspruch 1 angegebene Bedeutung besitzen, partiell oder vollständig zum ungesättigten oder gesättigten Alkohol hydriert oder reduziert und gegebenenfalls anschließend den erhaltenen gesättigten oder ungesättigten Alkohol in an sich bekannter Weise verestert.

**9.** Verwendung der Verbindungen nach Anspruch 1 bis 8 zur Verleihung, Verbesserung oder Modifizierung der Dufteigenschaften von Parfüms oder parfümierten Produkten.

**10.** Parfümkompositionen, enthaltend mindestens eine der Verbindungen gemäß den Ansprüchen 1 bis 8.

## Claims

**1.** An aliphatic alcohol or ester of the formula I

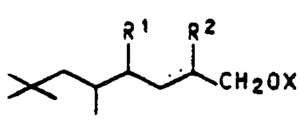

(I)

where $R^1$ is hydrogen or methyl, $R^2$ is straight-chain or branched C1-C4-alkyl and X is hydrogen or acyl of 2 to 4 carbon atoms and the dotted line is a further C-C bond.

**2.** A compound as claimed in claim 1, wherein X is acetyl or propionyl.

**3.** 2,5,7,7-Tetramethyloctanol

**4.** 2,5,7,7-Tetramethyloctyl acetate

**5.** 2,5,7,7-Tetramethyloctyl propionate

**6.** 2,5,7,7-Tetramethyloct-2-en-1-ol

7. 2,5,7,7-Tetramethyloct-2-en-1-yl acetate

8. A process for the preparation of an aliphatic alcohol or ester of the formula I, wherein an octenal or octanal of the formula II

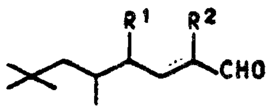

(II)

where $R^1$ and $R^2$ have the meanings stated in claim 1, are partially or completely hydrogenated to give the unsaturated or saturated alcohol and, if required, the saturated or unsaturated alcohol contained is then esterified in a conventional manner.

9. Use of a compound as claimed in any of claims 1 to 8 for imparting fragrance properties to, or improving or modifying the fragrance properties of, perfumes or perfumed products.

10. A perfume composition containing one or more of the compounds as claimed in any of claims 1 to 8.

**Revendications**

1. Alcools et esters aliphatiques de formule générale I

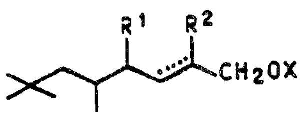

(I)

dans laquelle
- $R^1$ est mis pour un atome d'hydrogène ou un groupement méthyle,
- $R^2$ est mis pour un reste alkyle en $C_1$ à $C_4$ à chaîne droite ou ramifiée,
- X est mis pour un atome d'hydrogène ou un groupement acyle à 2-4 atomes de carbone, et la ligne en pointillé est mise pour une liaison C-C supplémentaire.

2. Composés selon la revendication 1, caractérisés en ce que X est mis pour un groupement acétyle ou propionyle.

3. 2,5,7,7-Tétraméthyl-octanol.

4. Acétate de 2,5,7,7-tétraméthyl-octyle.

5. Propionate de 2,5,7,7-tétraméthyl-octyle.

6. 2,5,7,7-Tétraméthyl-2-octène-1-ol.

7. Acétate de 2,5,7,7-tétraméthyl-2-octène-1-yle.

8. Procédé de préparation d'alcools et esters aliphatiques de formule générale I, caractérisé en ce qu'on hydrogène ou an réduit partiellement ou complètement un octènal ou un octanal de formule générale II

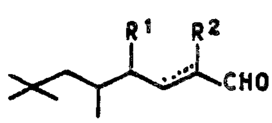

(II)

dans laquelle $R^1$ et $R^2$ ont les significations données dans la revendication 1, pour obtenir l'alcool insaturé ou saturé, puis on estérifie éventuellement de façon connue en soi l'alcool saturé ou insaturé obtenu.

**9.** Utilisation des composés selon l'une quelconque des revendications 1 à 8 pour créer, améliorer ou modifier les caractéristiques odoriférantes de parfums ou de produits de parfumerie.

**10.** Compositions de parfums, contenant au moins un des composés selon l'une quelconque des revendications 1 à 8.